# EUROPEAN PATENT APPLICATION

(11) **EP 1 938 698 A1**
(43) Date of publication of application: **02.07.2008**
(21) Application number: 06025262.4
(22) Date of filing: 06.12.2006
(51) Int. Cl.: A23L 1/30, A23L 1/212, C05F 5/00

(54) **Novel powders based on vegetation water from olive oil production**

(71) Applicant: DSM IP Assets B.V., 6411 TE Heerlen (NL)
(72) Inventor: The designation of the inventor has not yet been filed
(74) Representative: Steck, Melanie

(57) **Abstract**

The present invention is directed a process for the manufacture of a powder comprising vegetation water rich in water-soluble polyphenols, especially rich in hydroxytyrosol, as well as to the powders themselves and dietary supplements, especially in form of tablets, food, feed and cosmetic preparations containing such powders.

The process comprises the following steps:
A) providing vegetation water (concentrate) containing water-soluble polyphenols;
B) (optionally) modifying the water content of the vegetation water (concentrate);
C) optionally centrifuging the vegetation water (concentrate) and separating the water-soluble part;
D) spray-drying or granulating the (water-soluble part of the) vegetation water (concentrate), preferably in presence of an adjuvant;
E) optionally washing the thus obtained powder comprising vegetation water rich in water-soluble polyphenols with an organic solvent.

## Description

The present invention is directed to a process for the manufacture of a powder comprising olive vegetation water rich in water-soluble polyphenols, especially rich in hydroxytyrosol, as well as to the powders themselves and dietary supplements, especially in form of tablets, food, feed and cosmetic preparations for humans containing such powders.

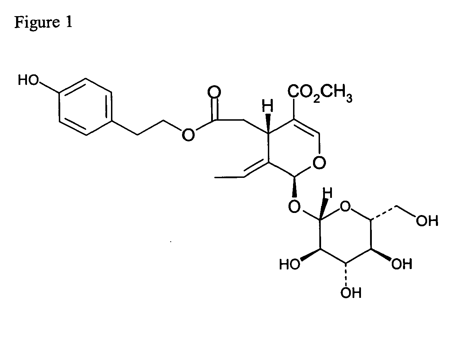

Vegetation water, a by-product of the olive oil production, has several beneficial biological effects such as antioxidant, heart health promoting, cancer preventive and antiinflammatory properties due to its content of water-soluble polyphenols such as hydroxytyrosol (2-(3,4-dihydroxyphenyl)ethanol) and oleuropein (shown in Fig. 1).

In most cases powders manufactured from vegetation water have a limited flowability due to the still high content of oil and fibers. A low flowability is disadvantageous for further processing of the powder, may it be the production of tablets or the production of food fortified with such powder. A good flowability, however, eases the handling of such powder. Therefore, the object of the present invention is to provide a powder with better flowability properties than the powders that are at present commercially available. A further object of the present invention is to provide an economic process for manufacturing such a powder.

The need for such a powder and such a process is met by the present invention.

Thus, the present invention is directed to a process for the manufacture of a powder comprising vegetation water rich in water-soluble polyphenols comprising the following steps:
A) providing vegetation water (or vegetation water concentrate) containing water-soluble polyphenols;
B) (optionally) modifying the water content of the vegetation water (or vegetation water concentrate);
C) optionally centrifuging the vegetation water (concentrate) and separating the water-soluble part;
D) spray-drying or granulating the (water-soluble part of the) vegetation water (concentrate), preferably in presence of an adjuvant;
E) optionally washing the thus obtained powder comprising vegetation water rich in water-soluble polyphenols with an organic solvent.

The water-soluble polyphenol is preferably selected from the group consisting of hydroxytyrosol, oleuropein, tyrosol and mixtures thereof. "Rich in water-soluble polyphenols" in the context of the present invention means that the amount of the water-soluble polyphenols is ≥ 0.1 weight-%, preferably ≥ 0.3 weight-%, more preferably ≥ 0.5 weight-%, most preferably ≥ 30 weight-%, based on the original vegetation water as used as starting material in the process.

The process is especially one for the manufacture of a powder comprising vegetation water rich in hydroxytyrosol. "Rich in hydroxytyrosol" in the context of the present invention means that the amount of hydroxytyrosol is ≥ 0.5 weight-%, preferably ≥ 1 weight-%, more preferably ≥ 2.5 weight-%, most preferably ≥ 25 weight-%,based on the dry weight of the original vegetation water.

The powder comprising vegetation water "rich in water-soluble polyphenols/hydroxyl-tyrosol" in the context of the present invention can also mean powder where the amount of the water-soluble polyphenols/hydroxytyrosol is ≥ 25% weight-%, preferably ≥ 50% weight-%, more preferably ≥ 70 weight-%, most preferably ≥ 90 weight-%, based on the total weight of the powder.

Each step is described in more detail in the following. The order of the process steps can be, but is not necessarily as listed here.

### Step A)

Vegetation water suitable as starting material for the process of the present invention may be any vegetation water produced by a method known to the person skilled in the art. "Vegetation water" in the context of the present invention encompasses any kind of olive waste water, olive juice and aqueous fractions of olives.

In further embodiments of the present invention not the vegetation water itself is used but its concentrate with a low water content.

A "vegetation water concentrate" means here a vegetation water where most of the water has been removed by any method known to the person skilled in the art may it be freeze-drying or distilling off the water (preferably under reduced pressure). A "low water content" in this respect means an amount of water below 6 weight-%, preferably below 5 weight-%, more preferably below 3 weight-%, based on the total weight of the vegetation water concentrate.

The vegetation water (concentrate) used in the present invention may especially have been manufactured according to one of the processes disclosed in US 6,416,808 (column 4, line 37 to column 7, line 27); WO 2004/005228; US 6,936,287; US 2005-103 711; US 2003-108 651; US 2002-198 415; US 6,165,475; JP 2001-252 054; JP 2000-319 161; WO 01/45514 (Usana); US 6,358,542 (see especially column 4, line 1 to column 9, line 50 and examples 1-5 and 11-13); US 6,361,803 (see especially column 3, line 64 to column 9, line 47 and examples 1-5 and 11-13); and WO 2006/084 658.

In preferred embodiments of the present invention the vegetation water (concentrate) was manufactured as disclosed in US 6,416,808 (column 4, line 37 to column 7, line 27).

An especially suitable vegetation water concentrate is e.g. "HIDROX® 6%", commercially available from CreAgri, Hayward, USA. "HIDROX® 6%" contains 5 to 8 weight-% of proteins, 45 to 68 weight-% of carbohydrates, 17 to 30 weight-% of fat, 8 to 15 weight-% of ash and a minimum of 6 weight-% of water-soluble simple and polyphenols, based on the total weight of HIDROX® 6%.

"HIDROX® 2%" and "HIDROX® 9%", both also commercially available from CreAgri, Hayward, USA, may also be used, as well as the following products commercially available from Glanbia and Indena (Milan, Italy): OLIVACTIV^{™} containing from 20 to 35 weight-% of hydroxytyrosol and from 4 to 6 weight-% of tyrosol; OLEASELECT^{™} having a total content of phenols of ≥ 30 weight-% (measured by UV) and an amount of hydroxytyrosol of ≥ 1.5 weight-% (measured by HPLC) and an amount of verbascoside of ≥ 5.0 weight-% (measured by HPLC) and OLIVE(OLEA)DRY, a powder containing from 22 to 24 g of hydroxytyrosol and from 5.0 to 6.5 g of tyrosol per kg.

Further suitable products are Prolivols, commercially available from Seppic, containing 35 weight-% of polyphenols, especially 20 mg hydroxytyrosol (per g of Prolivols) and 3 mg of tyrosol (per g of Prolivols); as well as Olive Braun Standard 500 (from obipektin): a powder containing from 1.0 to 2.2 g of hydroxytyrosol and from 0.2 to 0.7 g of tyrosol per kg; Olivex olive polyphenol liquid P10 (from Albert Isliker): a liquid containing from 2.0 to 3.5 g of hydroxytyrosol and from 0.2 to 1.0 g of tyrosol per kg; Olivex olive polyphenol (from Albert Isliker): a powder containing from 22 to 23 g of hydroxytyrosol and from 6.5 to 8.0 g of tyrosol per kg; and Olive Polyphenols NLT (from Lalilab Inc.) containing from 2.0 to 6 weight-% of hydroxytyrosol and from 0.7 to 1.1 weight-% of tyrosol.

In suitable commercially available vegetation water concentrates the amount of hydroxytyrosol varies in the range of from 1.0 to 220 g per kg of the total weight of the vegetation water concentrate. The amount of tyrosol preferably varies in the range of from 0.2 to 45 g per kg of the total weight of the vegetation water concentrate. The weight ratio of hydroxytyrosol to tyrosol is preferably between 100 : 10 and 100 : 40, most preferably between 100 : 18 and 100 : 35.

An especially preferred hydroxytyrosol-rich vegetation water (concentrate) for use in the process of the present invention is manufactured by a process comprising the following steps:
i) producing vegetation water from olives;
ii) adding acid to the vegetation water thereby producing acidified vegetation water;
iii) incubating the acidified vegetation water for a period until at least 50% of oleuropein originally present in the vegetation water has been converted to hydroxytyrosol;
iv) optionally removing (part of) the water of the thus obtained hydroxytyrosol-rich vegetation water, e.g. by freeze-drying.

### Step i)

Preferably the vegetation water is produced from the meat or pulp of depitted olives.

Preferably the vegetation water is obtained by the following steps:
optionally separating olive pits form olives to obtain a pitless olive pulp;
pressing the (pitless) olive pulp to obtain a liquid-phase mixture including water, oil, and olive pulp components;
separating the water component from the oil and olive pulp components of the liquid-phase mixture to obtain a water component substantially free of oil and olive pulp;
collecting the separated water component.

### Step ii)

Preferably the acid is added in an amount effective to produce a pH in the range of from 1 to 6, preferably in the range of from 3 to 5.

The acid is preferably selected from the group consisting of any organic and inorganic acids suitable for the human consumption (e.g. tartaric acid, lactic acid, citric acid, malic acid, phosphoric acid). More preferably citric acid and/or phosphoric acid is/are used.

### Step iii)

Preferably the acidified vegetation water is incubated for a period until at least 75% of oleuropein originally present in the vegetation water has been converted to hydroxytyrosol.

### Step B)

In step B) so much water is added to a vegetation water concentrate or removed from vegetation water so that the amount of water in the resulting suspension is in the range of from 7 to 70% weight-% , more preferably in the range of from 20 to 60% weight-%, most preferably in the range of from 40 to 50 weight-%, based on the total weight of the resulting suspension. Then the concentration is optimal for spray-drying or granulating.

### Step C)

The fat-soluble part (oil) and the water-insoluble part are disregarded. The water-soluble part, where the water-soluble polyphenols, especially hydroxytyrosol, are enriched, is further used in step D).

### Step D)

Step D), the transformation of the vegetation water (concentrate) into a powder, is either performed by spray-drying or by granulating.

The spray-drying in step D) is preferably performed at an inlet temperature in the range of from 120 to 220°C, preferably in the range of from 130 to 210°C, more preferably in the range of from 130 to 150°C, and at an outlet-temperature in the range of from 75 to 90°C.

The granulating in step D) is carried out using standard granulation processes like fluid-bed granulation, extrusion granulation, and mixer granulation using binders such as modified starches, pectin, polysaccharides and proteins.

Preferably the spray-drying is performed in the presence of an adjuvant selected from the group consisting of monosaccharides, disaccharides, oligosaccharides and polysaccharides. More preferably the spray-drying is performed in the presence of maltodextrin, preferably with a dextrose equivalent of from 5 to 8. The addition of maltodextrin results in a significantly improved yield, better flowability, and lower hygroscopicity.

Preferred examples of mono- and disaccharides are sucrose, invert sugar, xylose, glucose, fructose, lactose, maltose, saccharose and sugar alcohols.

Preferred examples of the oligo- and polysaccharides are starch, modified starch and starch hydrolysates. Preferred examples of starch hydrolysates are dextrins and maltodextrins, especially those having the range of 5 to 65 dextrose equivalents (DE), and glucose syrup, especially such having the range of 20 to 95 DE. The term "dextrose equivalent" (DE) denotes the degree of hydrolysis and is a measure of the amount of reducing sugar calculated as D-glucose based on dry weight; the scale is based on native starch having a DE close to 0 and glucose having a DE of 100.

A preferred modified polysaccharide is a modified starch. Starches are hydrophilic and therefore do not have emulsifying capacities. However, modified starches are made from starches substituted by known chemical methods with hydrophobic moieties. For example starch may be treated with cyclic dicarboxylic acid anhydrides such as succinic anhydrides, substituted with a hydrocarbon chain (see O. B. Wurzburg (editor), "Modified Starches: Properties and Uses, CRC Press, Inc. Boca Raton, Florida, 1986 (and subsequent editions). A particularly preferred modified starch of this invention has the following formula (I) 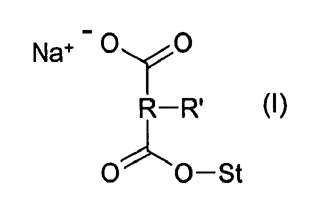 wherein St is a starch, R is an alkylene radical and R' is a hydrophobic group. Preferably R is a lower alkylene radical such as dimethylene or trimethylene. R' may be an alkyl or alkenyl group, preferably having 5 to 18 carbon atoms. A preferred compound of formula (I) is an "OSA-starch" (starch sodium octenyl succinate). The degree of substitution, i.e. the number of esterified hydroxyl groups to the number of free non-esterified hydroxyl groups usually varies in a range of from 0.1 % to 10%, preferably in a range of from 0.5% to 4%, more preferably in a range of from 3% to 4%.

The term "OSA-starch" denotes any starch (from any natural source such as corn, wheat, tapioca and potatoe or synthesized) that was treated with octenyl succinic anhydride (OSA). The degree of substitution, i.e. the number of hydroxyl groups esterified with OSA to the number of free non-esterified hydroxyl groups usually varies in a range of from 0.1% to 10%, preferably in a range of from 0.5% to 4%, more preferably in a range of from 3% to 4%. OSA-starches are also known under the expression "modified food starch".

The term "OSA-starches" encompasses also such starches that are commercially available e.g. from National Starch under the tradenames HiCap 100, Capsul, Capsul HS, Purity Gum 2000, UNI-PURE, HYLON VII; from Roquette Frères; from CereStar under the tradename C*EmCap or from Tate & Lyle. These commercially available starches may also be suitable adjuvants.

### Step E)

The organic solvent used in step E) is preferably an organic solvent with a boiling point < 115°C.

The organic solvent is preferably selected from the group consisting of dichloromethane, n-hexane, acetone, toluene, ethanol, *iso*-propanol, ethyl acetate and mixtures thereof.

If necessary the powder washed with the organic solvent is further dried, e.g. in a dryer oven, in a fluid bed dryer, or continuous drying in a microwaves oven.

The present invention is also directed to a process for the manufacture of a powder comprising vegetation water rich in water-soluble polyphenols comprising the following steps:
A) providing vegetation water (or vegetation water concentrate) containing water-soluble polyphenols;
B) (optionally) modifying the water content of the vegetation water (or vegetation water concentrate);
C) optionally centrifuging the vegetation water (concentrate) and separating the water-soluble part;
D*) (spray-)drying the (water-soluble part of the) vegetation water (concentrate);
E) washing the thus obtained powder comprising vegetation water rich in water-soluble polyphenols with an organic solvent.

Details and preferences of steps A), B), C) and E) are as described above. Step D*) is preferably carried out as described for step D).

A special embodiment of the present invention is a process for the manufacture of a powder comprising vegetation water rich in hydroxytyrosol comprising the following steps:
a) producing vegetation water from olives (see step A) and step i) above);
b) adding acid to the vegetation water thereby producing acidified vegetation water; (see step ii) above)
c) incubating the acidified vegetation water for a period until at least 50% of oleuropein originally present in the vegetation water has been converted to hydroxytyrosol; (see step iii) above)
d) optionally concentrating the thus obtained hydroxytyrosol-rich vegetation water by removing (part of) the water; (see step B) above)
e) optionally freeze-drying the thus obtained concentrated hydroxytyrosol-rich vegetation water;
f) optionally re-suspending the freeze-dried hydroxytyrosol-rich vegetation water concentrate in water;
g) optionally centrifuging the vegetation water concentrate and separating the water-soluble part; (see step C) above)
h) spray-drying or granulating the (water-soluble part of the) vegetation water (concentrate), preferably in presence of an adjuvant; (see step D) above)
j) optionally washing the thus obtained powder comprising vegetation water rich in hydroxytyrosol with an organic solvent. (see step E) above)

The present invention is also directed to the powders (comprising vegetation water rich in water-soluble polyphenols, especially rich in hydroxytyrosol) themselves manufactured according to the processes of the present invention disclosed above.

The powders manufactured according to the processes of the present invention have a good flowability. Thus, they are especially suitable for the manufacture of (coated/uncoated) tablets containing them. Therefore, the present invention is also directed to tablets comprising such powders comprising vegetation water rich in water-soluble polyphenols manufactured by any one of the processes of the present invention.

The further processing of the powders according to the present invention is not restricted to tablets. Due to the good flowability of the powders of the present invention their handling is facilitated in general. So they may also be used for the manufacture of (other) galenic forms as mentioned below.

Thus, the present invention is also directed to a dietary supplement comprising a powder comprising vegetation water rich in water-soluble polyphenols manufactured by any one of the processes of the present invention.

Galenic forms that are suitable for administration to the human body, especially for oral administration, are pills, granules, dragées, capsules, and effervescent formulations such as effervescent powders and effervescent tablets. Dietary supplements or pharmaceutical compositions may not only be administered in solid form but also in liquid form such as in form of solutions, emulsions or suspensions as e.g. beverages, pastes and oily suspensions. The pastes may be filled into hard or soft shell capsules. Examples for other application forms are forms for transdermal, parenteral or injectable administration. The dietary and pharmaceutical compositions may also be in the form of controlled (delayed) release formulations.

The dietary compositions or pharmaceutical compositions according to the present invention may further contain protective hydrocolloids, binders, film forming agents, encapsulating agents/materials, wall/shell materials, matrix compounds, coatings, emulsifiers, surface active agents, solubilizing agents or disintegrants, adsorbents, carriers, fillers, co-compounds, dispersing agents, wetting agents, processing aids, flowing agents, weighting agents, jellyfying agents, gel forming agents, antioxidants and antimicrobials.

Since the powders of the present invention have also superior / improved organoleptic properties they are not only suitable for dietary supplements (and also for pharmaceutical compositions) but also for food consumed by humans. Thus, the present invention is also directed to food comprising a powder comprising vegetation water rich in water-soluble polyphenols (especially rich in hydroxytyrosol) manufactured by any one of the processes of the present invention.

The term "food" comprises any type of nutrition including also clinical nutrition, as well as food additives and beverages.

In further embodiments of the present invention the powders comprising vegetation water rich in water-soluble polyphenols (especially rich in hydroxytyrosol) manufactured by any one of the processes of the present invention are used as ingredient for cosmetic preparations.

Since the processes of the present invention improve the flowability of (powders comprising) vegetation water concentrates the present invention is also directed to a process for improving the flowability of vegetation water concentrates comprising the following steps:
optionally centrifuging the vegetation water concentrat) and separating the water-soluble part;
spray-drying or granulating the (water-soluble part of the) vegetation water concentrate, preferably in presence of an adjuvant; and
optionally washing the thus obtained powder comprising vegetation water rich in water-soluble polyphenols with an organic solvent.

For these steps the same preferences are applicable as described above.

The invention is now further illustrated by the following non-limiting examples.

### Examples

### Example 1

200 g of HIDROX 6% freeze dried powder were placed in a 1000 ml double wall vessel. 300 ml of de-ionized water were added and the mixture was brought into solution while stirring with a micer disc at 800 revolutions/minute (rpm) at ambient temperature. Thereupon the rpm of the micer disc was increased to 4800 rpm to emulsify the oil during 30 minutes. The solution was diluted with 300 ml de-ionized water to adjust the viscosity before spray drying.

The solution was spray dried on a lab spray dryer (Mobilie Minor^{™} 2000 Typ D1 from Niro) under following conditions:

| | |
|---|---|
| Inlet temperature | approx. 200°C |
| Outlet temperature | 78 - 86°C |
| Nozzle pressure | 4 bar (Speed: approx. 23'000 rpm) |
| Spray rate | approx. 2.5 kg/hour |
| Yield | 54 g (27% of the theoretical amount) |

The analytical data are given in table 1.

### Example 2

100 g of HIDROX 6% freeze dried powder and 100 g maltodextrin DE-0508 were placed in a 1000 ml double wall vessel and processed analogue example 1.
Yield 132 g (66% of the theoretical amount).

The analytical data are given in table 1.

### Example 3: Extraction of vegetation water concentrates with ethyl acetate

10 x 5 g of HIDROX 6% freeze dried powder were placed in 50 ml centrifuge tubes. 40 ml of ethyl acetate were added and the centrifuge tubes were shaked for 1 hour on a shaker (IKA HS 501 D) with 300 motion/minute to dissolve the oily soluble ingredients. Afterwards the solid particles were filtered using a round filter and suction bottle. The solid particles were washed again 3 times with 50 ml of ethyl acetate.

The washed material has been dried at ambient temperature on the filter using the suction bottle under vacuum and afterwards for one hour in a drying oven.

The ethyl acetate phase containing the oily ingredients has been evaporated on a rotary evaporator.

The analytical data of the dry powder resulting from the evaporation of the ethyl acetate phase containing the hydroxytyrosol are summarized in table 1.

### Example 4: Centrifugation and Spray-Dryins of vegetation water

12 x 12.5 g (total 150 g) of HIDROX 6% freeze dried powder were placed in 50 centrifuge tubes. 34 ml of de-ionized water were added and the centrifuge tubes were shaked for 1 hour on a shaker (IKA HS 501 D) with 300 motion/minute to re-disperse the powder. Thereof, they were centrifuged for 6 hours at 3700 rpm (Sigma 6-10). The oil phase on the top and the "solid" phase on the bottom were removed and the water phase was collected. The water phase (412 g) containing 77.9 g dry substance was spray dried on the lab spray dryer Mobile Minor 2000 from Niro under following conditions:

| | |
|---|---|
| Inlet temperature | approx. 140°C |
| Outlet temperature | 76 - 82°C |
| Nozzle pressure | 4 bar (Speed: approx. 23'000 rpm) |
| Spray rate | approx. 0.8 kg/hour |

Yield 6 g (7.7% of the theoretical amount).

The dry substance content in the water phase was 18.9 weight-% (77.9 g dry substance, approx. 52 weight-% of the total used material).

The analytical data are given in table 1.

### Example 5: Centrifugation and Spray-Drying of vegetation water by using maltodextrin

The water soluble part was prepared as described in example 4. To improve yield and flowability 63.6 g of maltodextrin were added to 344 g water phase containing 63.6 g dry substance before spray drying. The aqueous solution was spray dried in an analogue way as described in example 4.
Yield 107 g (86% of the theoretical amount).

The analytical data are summarized in table 1.

**Table 1: HYDROX reworked dry powders: analytical data in comparison to the current sales product**

| Description | HIDROX 6% Freeze Dried Powder | HIDROX 6% Spray Dried Powder | HIDROX 3% Spray Dried Powder (diluted with Maltodextrin) | HIDROX 6% (washed with ethyl acetate)¹⁾ | HIDROX 6% Spray Dried Powder (water soluble part after centrifugation) | HIDROX 3% Spray Dried Powder (water soluble part after centrifugation, addition of maltodextrin) |
|---|---|---|---|---|---|---|
| Hydroxytyrosol content | 2.0 % | 1.9 % | 1.1 % | 2.4 % | 3.0 %²⁾ | 1.7 % |
| Loss on drying (105°C) | 3.1 % | 4.2 % | 3.0 % | 3.8 % | 2.5 % | 2.5 % |
| Total oil content (grav.) | 17.3 % | 17.8 % | 9.8 % | 5.6 % | <1% | 0.34% |
| Surface, extractable oil content (grav.) | 14.1 % | 4.6 % | 1.7 % | 0.24 % | 0.7 % | 0.08 % |
| - as-is | 81.5 % | 25.8 % | 17.3% | 4.3 % | ~ 100% | 23.5 % |
| - related to total oil content | | | | | | |

| | | | | | | |
|---|---|---|---|---|---|---|
| ¹⁾ The residual oil after washing with ethyl acetate contains 0.14% hydroxytyrosol. Good flowability. ²⁾ approx. 79% of the theoretical amount was found in the spray dried powder | | | | | | |

## Claims

1. A process for the manufacture of a powder comprising vegetation water rich in water-soluble polyphenols comprising the following steps:
A) providing vegetation water (concentrate) containing water-soluble polyphenols;
B) (optionally) modifying the water content of the vegetation water (concentrate);
C) optionally centrifuging the vegetation water (concentrate) and separating the water-soluble part;
D) spray-drying or granulating the (water-soluble part of the) vegetation water (concentrate), preferably in presence of an adjuvant;
E) optionally washing the thus obtained powder comprising vegetation water rich in water-soluble polyphenols with an organic solvent.

2. The process according to claim 1, wherein in step B) so much water is added or removed so that the amount of water in the resulting suspension is in the range of from 7 to 70% weight-% , more preferably in the range of from 20 to 60% weight-%, most preferably in the range of from 40 to 50 weight-%, based on the total weight of the resulting suspension.

3. The process according to claim 1 or 2, wherein the water-soluble polyphenol is selected from the group consisting of hydroxytyrosol, oleuropein, tyrosol and mixtures thereof.

4. The process according to any of the preceding claims, wherein the spray-drying in step D) is performed at an inlet temperature in the range of from 120 to 220°C and at an outlet-temperature in the range of from 75 to 90°C.

5. The process according to any of the preceding claims, wherein the adjuvant used in step D) is selected from the group consisting of monosaccharides, disaccharides, oligosaccharides, polysaccharides and mixtures thereof.

6. The process according to any of the preceding claims, wherein the organic solvent is selected from the group consisting of dichloromethane, n-hexane, acetone, toluene, ethanol, *iso*-propanol, ethyl acetate and mixtures thereof.

7. The process according to any of the preceding claims, wherein in step C) the fat-soluble and the water-insoluble parts of the vegetation water are removed.

8. A process for the manufacture of a hydroxytyrosol-rich vegetation water (concentrate) comprising the following steps:
i) producing vegetation water from olives;
ii) adding acid to the vegetation water thereby producing acidified vegetation water;
iii) incubating the acidified vegetation water for a period until at least 50% of oleuropein originally present in the vegetation water has been converted to hydroxytyrosol;
iv) optionally removing (part of) the water of the thus obtained hydroxytyrosol-rich vegetation water, e.g. by freeze-drying.

9. The process according to claim 8, wherein the vegetation water is produced from the meat or pulp of depitted olives.

10. The process according to claim 8 or 9, wherein the incubating is carried out until the vegetation water has a weight ratio of hydroxytyrosol to oleuropein in the range of from 1 : 1 to 200 : 1, preferably in the range of from 4 : 1 to 200 : 1, more preferably in the range of from 10 : 1 to 100 : 1.

11. The process according to any one or more of claims 8 to 10, wherein the incubating is carried out until the vegetation water has a weight ratio of hydroxytyrosol and tyrosol in the range of from 3 : 1 to 50 : 1, preferably in the range of from 5 : 1 to 30 : 1.

12. The process according to any one or more of claims 8 to 11, wherein said acid is added in an amount effective to produce a pH in the range of from 1 to 6, preferably in the range of from 3 to 5.

13. The process according to any one or more of claims 8 to 12, wherein said acid is citric acid or phosphoric acid.

14. The process according to any one or more of claims 8 to 13 wherein step a) is performed by the following steps:
optionally separating olive pits from olives to obtain a pitless olive pulp;
pressing the (pitless) olive pulp to obtain a liquid-phase mixture including water, oil, and olive pulp components;
separating the water component from the oil and olive pulp components of the liquid-phase mixture to obtain a water component substantially free of oil and olive pulp;
collecting the separated water component.

15. A process for the manufacture of a powder comprising vegetation water rich in hydroxytyrosol comprising the following steps:
a) producing vegetation water from olives;
b) adding acid to the vegetation water thereby producing acidified vegetation water;
c) incubating the acidified vegetation water for a period until at least 50% of oleuropein originally present in the vegetation water has been converted to hydroxytyrosol;
d) optionally concentrating the thus obtained hydroxytyrosol-rich vegetation water by removing (part of) the water;
e) optionally freeze-drying the thus obtained concentrated hydroxytyrosol-rich vegetation water;
f) optionally re-suspending the freeze-dried hydroxytyrosol-rich vegetation water concentrate in water;
g) optionally centrifuging the vegetation water concentrate and separating the water-soluble part;
h) spray-drying or granulating the (water-soluble part of the) vegetation water (concentrate), preferably in presence of an adjuvant;
j) optionally washing the thus obtained powder comprising vegetation water rich in hydroxytyrosol with an organic solvent.

16. A tablet comprising a powder comprising vegetation water rich in water-soluble polyphenols manufactured by any one of the preceding claims.

17. A dietary supplement comprising a powder comprising vegetation water rich in water-soluble polyphenols manufactured by any one of the preceding claims.

18. Food or feed comprising a powder comprising vegetation water rich in water-soluble polyphenols manufactured by any one of the preceding claims.

19. A cosmetic preparation comprising a powder comprising vegetation water rich in water-soluble polyphenols manufactured by any one of the preceding claims.

20. A process for improving the flowability of vegetation water concentrates comprising the following steps:
optionally centrifuging the vegetation water (concentrate) and separating the water-soluble part;
spray-drying or granulating the (water-soluble part of the) vegetation water (concentrate), preferably in presence of an adjuvant; and
optionally washing the thus obtained powder comprising vegetation water rich in water-soluble polyphenols with an organic solvent.
